# EUROPÄISCHE PATENTSCHRIFT

(11) **EP 2 411 438 B1**
(45) Veröffentlichungstag und Bekanntmachung des Hinweises auf die Patenterteilung: **06.03.2013**
(21) Anmeldenummer: 10710229.5
(22) Anmeldetag: 19.03.2010
(51) Int. Cl.: C08G 18/78, C08G 18/24, C07C 273/18

(54) **HERSTELLUNG VON POLYISOCYANAT-PREPOLYMEREN MIT ALLOPHANAT-STRUKTUREINHEITEN UND DEREN VERWENDUNG IN FORMULIERUNGEN FÜR BESCHICHTUNGEN, KLEBSTOFFE UND DICHTSTOFFE**
PRODUCTION OF POLYISOCYANATE PREPOLYMERS HAVING ALLOPHANATE STRUCTURE UNITS AND USE THEREOF IN FORMULATIONS FOR COATINGS, ADHESIVES AND SEALANTS
PRODUCTION DE PRÉPOLYMÈRES DE POLYISOCYANATE À MOTIFS STRUCTURAUX ALLOPHANATE ET LEUR UTILISATION DANS DES FORMULATIONS POUR ENDUITS, ADHÉSIFS ET PRODUITS D'ÉTANCHÉITÉ

(30) Priorität: 27.03.2009 DE 102009014676
(43) Veröffentlichungstag der Anmeldung: 01.02.2012
(73) Patentinhaber: Bayer MaterialScience AG, 51368 Leverkusen (DE)
(72) Erfinder: PFEIFFER, Evelyn, 51375 Leverkusen (DE); KARAFILIDIS, Christos, 51375 Leverkusen (DE); SCHMIDT, Axel, 239191 Singapore (SG); REICHERT, Peter, 41541 Dormagen (DE)
(86) Internationale Anmeldenummer: PCT/EP2010/001748
(87) Internationale Veröffentlichungsnummer: WO 2010/108632

(56) Entgegenhaltungen:
- WO-A1-2005/097865
- DE-A1-102004 015 985
- GB-A- 994 890

## Beschreibung

Die Erfindung betrifft die Verwendung und Herstellung von Polyisocyanat-Prepolymeren mit Allophanat-Struktureinheiten in Formulierungen für Beschichtungen, Klebstoffe und Dichtstoffe.

Polyisocyanat-Prepolymere mit Allophanat-Struktureinheiten sind insbesondere deshalb interessant, weil sie bei vergleichsweise niedriger Viskosität einen hohen NCO-Gehalt aufweisen. Sie stellen wertvolle Vernetzer für zweikomponentige Polyurethansysteme dar, mit blockierten NCO-Gruppen können sie darüber hinaus auch in Einkomponenten-Polyurethansystemen eingesetzt werden.

Polyisocyanat-Prepolymere mit Allophanat-Struktureinheiten sind prinzipiell bekannt.

In EP-A 0 303 150 wurde z.B. ein Verfahren zur Herstellung aliphatischer Allophanate beschrieben, welches bei hohen Temperaturen (> 200°C) ohne die Verwendung von Katalysatoren durchgeführt wird. Die notwendige rasche Erwärmung und das Abkühlen sind jedoch in der Praxis, d.h. bei großen Ansätzen, kaum umsetzbar.

In EP-A 0 712 840 wird die Verwendung von Zink-Verbindungen wie Zinkstearat, Zinkoctoat, Zinknaphtenat und Zinkacetylacetonat als Katalysatoren für die Allophanatisierung beschrieben. Bei diesem Verfahren werden allerdings NCO- und OH-gruppenfreie Urethane für die Allophanatisierung eingesetzt. Dazu muss die Stöchiometrie der Reaktionspartner genau kontrolliert werden und Produkte mit einer NCO-Funktionalität wesentlich höher als 2 sind auf Basis dieses Verfahrens nicht oder nur sehr schwer möglich. Die vorliegende Erfindung erlaubt es hochfunktionelle Prepolymere mit vergleichsweise niedriger Viskosität und enorm hohem NCO-Gehalt herzustellen. Ferner muss die Stöchiometrie der Reaktion nicht kontrolliert werden, da nach der vorliegenden Erfindung ein großer Überschuss des Isocyanates verwendet werden kann. Darüber hinaus muss nach EP-A 0 712 840 das für die Herstellung der Urethane eingesetzte Polyisocyanat stets verschieden zu dem für die (nachfolgende) Allophanatisierung eingesetzten sein. Das hat in der großindustriellen Produktion den Nachteil, dass man einen zusätzlichen Lagertank und eine zusätzliche Dosiervorrichtung benötigt und man sicherstellen muss, dass die Urethanisierung vollständig abgelaufen ist, bevor man das zweite Polyisocyanat zudosiert. Die vorliegende Erfindung verwendet dagegen nur eine Isocyanat in hohem Überschuss. Daher ist es nicht notwendig den Reaktionsverlauf und die Stöchiometrie so genau zu kontrollieren und kann ohne ein zweites Dosieren eines Polyisocyanates die Allophanatisierungsreaktion starten.

In EP-A0 682 012 sind u.a. Prepolymere auf Basis von 1 bis 4 Hydroxylgruppen aufweisenden Polyethern und Diisocyanaten beschrieben, welche unter Verwendung von Zinn(II)-Verbindungen mit einem Überschuss der Diisocyanate zu den entsprechenden Allophanaten umgesetzt werden können. Allerdings können Zinn(II)-Verbindungen nur unzureichend deaktiviert werden, so dass die erhaltenen Produkte bei Lagerung eine Zunahme der Viskosität und eine Abnahme des NCO-Gehaltes zeigen. Ferner ist die Verwendung von Zinn-Verbindungen vor allem in lebensmittelnahen oder medizinischen Anwendungen zu vermeiden.

In der Publikation GB-A 994 890 werden allophanatgruppen enthaltende Polyurethan-Prepolymere beschrieben. Diese Prepolymere werden hergestellt durch die Reaktion von n Mol eines Diisocyanates mit einem Mol eines Urethangruppen enthaltenden Isocyanates der Strukturformel R^{II}(OOCNHR^{I}NCO)n. R^{II} ist dabei ein Mono- oder Polyol. Dabei wird das urethangruppen= enthaltenden Isocyanat durch Reaktion des Mono- oder Polyoles mit einem Diisocyanat und anschließender Isolierung des dabei entstanden urethangruppen-enthaltenden Isocyanates gewonnen. Dieser Isolationsschritt ist technisch aufwendig und wird in der vorliegenden Erfindung vermieden. Nach GB-A 994 890 wird im folgenden das urethangruppen-enthaltenden Isocyanat mit einem Diisocyanat in einem molaren Überschuss von bis zu 300 % versetzt. Aufgrund des vergleichsweise geringen molaren Überschusses findet während der Allophanatisierungsreaktion eine Kettenverlängerung und damit ein Molekulargewichtsaufbau statt, der die Viskosität des resultierenden Produktes erheblich erhöht. In der vorliegenden Erfindung wird die Kettenverlängerung durch einen mehr als 10-fachen molaren Überschuss des Diisocyanates bei der Allophanatisierung vermieden, was zu deutlich niederviskosen Produkten führt.

In DE-A 10 2004 015983 wird ein Verfahren zur Herstellung von Polyisocyanat-Prepolymeren mit Allophanat-Struktureinheiten durch Verwendung von Zink-Verbindungen als Katalysatoren beschrieben. Jedoch ist der erreichte NCO-Gehalt der so hergesellten Prepolymere vergleichweise niedrig. In der vorliegenden Erfindung werden bei ähnlichen Viskositäten deutlich höhere NCO-Gehalte erreicht.

US-A 4 738 991 beschreibt die Herstellung von allophanatgruppen enthaltenden Polyisocyanat-Prepolymeren für die Verwendung in Rezepturen für die Schaumstoffherstellung. Dabei wird der metallorganische Katalysator bereits während der Urethanisierungsreaktion zwischen den Polyisocyanaten und den Mono- oder Polyolen zugegeben. Anschließend wird die Allophanatisierung mit dem gleichen Katalysator durchgeführt. Es ist bekannt, dass metallorganische Katalysatoren nicht nur die Urethanisierungs- und Allophanatisierungsreaktion sondern auch die Trimerisierungsreaktion katalysieren. Nach dem Verfahren in US-A 4738991 hat der Katalysator eine höhere Verweilzeit mit dem Polyisocyanat, so dass der Anteil an unerwünschten Nebenreaktion deutlich höher ist, als in der vorliegenden Erfindung. In der vorliegenden Erfindung wird der Katalysator erst nach der Urethanisierung zugegeben, was zu weniger Nebenreaktion und damit zu deutlich niedrigeren Viskositäten führt. Auch wird in der vorliegenden Erfindung Isophthathaloyldichlorid (IPDC) zum Abstoppen des Katalysators verwendet. Gegenüber den in US-A 4738991 verwendeten Stoppern weißt Isophthaloyldichlorid eine deutlich geringere Toxizität auf.

In EP-A 0 000 194 wird die Herstellung von allophanatgruppen enthaltenden Prepolymeren in Gegenwart von starken Säuren beschrieben. Nachteilig ist hier, dass die Reakivität der Polyisocyanate aufgrund der hohen Säurekonzentration während der Allophanatisierung sehr niedrig ist. So benötigt die vollständige Allophanatisierung nach diesem Stand der Technik 8,5 Stunden und länger, während durch den Katalysatoreinsatz gemäß vorliegenden Erfindung die Reaktionszeit kürzer als zwei Stunden ist. Aufgrund der längeren Reaktorbelegungszeit nach EP-A 0 000 194 sind Produkte, die nach diesem Verfahren hergestellt werden, aufwendiger in der Produktion und erfordern einen höheren Energieeinsatz als nach dem Verfahren in der vorliegenden Erfindung.

Aufgabe der vorliegenden Erfindung war daher die Bereitstellung eines NCO-gruppen terminierten Hexamethylendiisocyanat-Prepolymeren für die Verwendung in Formulierungen für Deschichtungen, Klebstoffe und Dichtstoffe, das gleichzeitig eine hohe Funktionalität, einen hohen NCO-Gehalt und eine niedrige Viskosität bei gleichzeitig guter Lagerstabilität aufweist und durch einen einstufigen Prozess herzustellen ist in dem das urethangruppen-enthaltende Zwischenprodukt weder einer aufwendigen Stöchiometrickontrolle noch einer Isolierung des Zwischenproduktes bedarf.

Überraschend wurde gefunden, dass Hexamethylendiisocyanat-Prepolymere, die Allophanat-Struktureinheiten enthalten, diese Anforderungen erfüllen, wenn sie hergestellt werden, indem man zunächst ein urethangruppen-enthaltendes Hexamethylendiisocyanat-Prepolymer mit einem mehr als zehnfachen molaren NCO-Gruppen- zu OH-Gruppen-Überschuss an Hexamethylendiisocyanat herstellt und anschließend ohne Isolierung des urethangruppen enthaltenden Hexamethylendiisocyanat-Prepolymeren und mit dem bereits erwähnten Überschuss an Hexamethylendiisocyanat die Allophanatisierung mit Zink(II)-Verbindungen, bevorzugt Zinkalkanoaten, als Katalysatoren durchführt und anschließend den Katalysator mit Isophthaloyldichlorid deaktivieren.

Gegenstand der Erfindung ist daher ein Verfahren zur Herstellung von Hexamethylendiisocyanat-Prepolymeren mit Allophanat-Struktureinheiten, dadurch gekennzeichnet, dass
A) zunächst in Abwesenheit eines Katalysators in einem molaren NCO-Gruppen- zu Oll-Gruppen-Überschuss von 10:1 bis 20:1
   a) Hexamethylendiisocyanat mit
   b) einer oder mehreren Polyhydroxyverbindungen eines zahlenmittleren Molekulargewichts Mn von 100 bis 300 g/mol
      zu einem NCO-funktionellen Polyurethan-Prepolymer umgesetzt werden, und dessen Urethangruppen werden anschließend unter weiterer Umsetzung mit
   c) dem überschüssigen Hexamethylendiisocyanat und
   d) Zink(II)-octoat als Katalysator
      teilweise oder vollständig allophanatisiert, der Katalysator wird anschließend mit Isophthaloyldichlorid gestoppt, und durch eine abschließende Kurzwegdestillation wird der Restmonomergehalt des so erhaltenen Hexamethylendiisocyanat-Prepolymeren mit Allophanatstruktureinheiten auf 0,5 Gew.-% oder weniger abgesenkt, so dass
B) der NCO-Gehalt des Hexamethylendiisocyanat-Prepolymeren mit Allophanat-Struktureinheiten zwischen 15 und 24 Gew.-% NCO-Gruppen liegt und
C) die Viskosität des Hexamethylendiisocyanat-Prepolymeren mit Allophanat-Stuktureinheiten zwischen 500 und 4000 mPas bei 23 °C liegt.

Als Polyhydroxyverbindungen der Komponente b) können alle dem Fachmann bekannten Polyhydroxyverbindungen eingesetzt werden, welche bevorzugt eine mittlere OH-Funktionalität 1,5 aufweisen.

Dies können beispielsweise niedermolekulare Diole, Triole (z.B. Trimethylolpropan) und Tetraole (z.B. Pentaerythrit), Polyetherpolyole, Polyesterpolyole, Polycarbonatpolyole sowie Polythioetherpolyole sein. Bevorzugte Polyhydroxyverbindungen sind Substanzen der vorstehend genannten Art auf Polyetherbasis.

Diese Polyetherpolyole weisen zahlenmittlere Molekulargewichte Mₙ von 100 bis 300 g/mol auf.

Ferner besitzen sie bevorzugt eine mittlere OH-Funktionalität von >1,5, bevorzugt > 1,9 und besonders bevorzugt ≥ 1,95.

Die OH-Funktionalität dieser Polyether ist dabei kleiner als 6, bevorzugt kleiner als 4 und besonders bevorzugt kleiner als 3.

Solche Polyetherpolyole sind in an sich bekannter Weise durch Alkoxylierung von geeigneten Starter--Molekülen unter Basenkatalyse oder durch Einsatz von Doppelmetallcyanidverbindungen (DMC-Verbindungen) zugänglich.

Besonders geeignete Polyetherpolyole der Komponente b) sind solche der vorstchend genannten Art mit einem Gehalt an ungesättigten Endgruppen von kleiner oder gleich 0,02 Milliäquivalenten pro Gramm Polyol (meq/g), bevorzugt kleiner oder gleich 0,01 meq/g, besonders bevorzugt kleiner oder gleich 0,01 meq/g (Bestimmungsmethode ASTM D2849-69).

Solche Polyetherpolyole haben dabei eine besonders enge Molekulargewichtsverteilung, d.h. eine Polydispersität (PD = M_{w}/Mₙ) von 1,0 bis 1,5 und/oder eine OH-Funktionalität ≥ 1,9. Bevorzugt weisen die genannten Polyetherpolyole eine Polydispersität von 1,0 bis 1,5 und eine OH-Funktionalität von größer 1,9 auf, besonders bevorzugt größer oder gleich 1,95 auf.

Derartige Polyetherpolyole sind in an sich bekannter Weise durch Alkoxylierung von geeigneten Starter-Molekülen, insbesondere unter Verwendung von Doppelmetallcyanid-Katalysatoren (DMC-Katalyse) herstellbar. Dies ist z.B. in der US-A 5 158 922 (z.B. Beispiel 30) und EP-A 0 654 302 (S. 5, Z. 26 bis S. 6, Z. 32) beschrieben.

Geeignete Starter-Moleküle für die Herstellung von Polyetherpolyolen sind beispielsweise einfache, niedermolekulare Polyole, Wasser, organische Polyamine mit mindestens zwei N-H-Bindungen oder beliebige Gemische derartiger Starter-Moleküle. Für die Alkoxylierung geeignete Alkylenoxide sind insbesondere Ethylenoxid und/oder Propylenoxid, die in beliebiger Reihenfolge oder auch im Gemisch bei der Alkoxylierung eingesetzt werden können.

Bevorzugte Starter-Moleküle zur Herstellung von Polyetherpolyolen durch Alkoxylierung, insbesondere nach dem DMC-Verfahren, sind einfache Polyole wie Ethylenglykol, Propylenglykol-1,3- und Butandiol-1,4, Hexandiol-1,6, Neopentylglykol, 2-Ethylhexandiol-1,3, Glyzerin, Trimethylolpropan, Pentaerythrit sowie niedermolekulare, Hydroxylgruppen aufweisende Ester derartiger Polyole mit Dicarbonsäuren oder niedermolekulare Ethoxylierungs- oder Propoxylierungsprodukte derartiger einfacher Polyole oder beliebige Gemische derartiger Polyhydroxyverbindungen.

Die Herstellung der isocyanatgruppenhaltigen Polyurethan-Prepolymere erfolgt durch Umsetzung der Polyhydroxyverbindungen der Komponente b) mit überschüssigen Mengen Hexamethylendiisocyanat aus a). Die Umsetzung erfolgt im Allgemeinen bei Temperaturen von 20 bis 140 °C, bevorzugt bei 40 bis 100 °C.

Die Allophanatisierung erfolgt dann anschließend durch Umsetzung der isocyanatgruppenhaltigen Polyurethan-Prepolymere mit Hexamethylendiisocyanat aus c), wobei als geeigneter Katalysatoren d) Zink(II)-octoat zur Allophanatisierung zugesetzt wird. Anschließend wird zur Stabilisierung IPDC zugesetzt, bevor überschüssiges Hexamethylendiisocyanat z.B. durch Dünnschichtdestillation, Umkristallisation oder Extraktion aus dem Produkt entfernt wird.

Das Molverhältnis der OH-Gruppen der Verbindungen der Komponente b) zu den NCO-Gruppen des Hexamethylendiisocyanates aus a) und c) beträgt 1 : 10 bis 1 : 20.

Als Katalysator d) wird Zink(II)-octoat eingesetzt,

Diese Allophanatisierungskatalysator wird typiseherweise in Mengen von bis zu 5 Gew.%, bezogen auf die gesamte Reaktionsmischung, eingesetzt. Bevorzugt werden 5 bis 500 ppm des Katalysators, besonders bevorzugt 20 bis 200 ppm, eingesetzt.

Der Zusatz von IPDC verbessert über die Deaktivierung des Allophanatisierungskatalysators hinaus die Stabilität der erfindungsgemäß hergestellte Allophanate, z.B. bei thermischer Belastung während der Dünnschichtdestillation oder auch nach der Herstellung bei Lagerung der Produkte.

Die sauren Additive werden in der Regel mindestens in einer solche Menge zugegeben, dass das Molverhältnis der sauren Zentren des sauren Additivs zu den aktiven Zentren des Katalysators mindestens 1:1 beträgt. Vorzugsweise wird jedoch ein Überschuss des sauren Additivs zugesetzt.

Der Restmonomitrengehalt des Hexamethylendiisocyanat-Prepolymeren mit Allophanat-Struktureinheiten wird durch eine abschließende Kurzwegdestillation auf 0,5 Ges.-% oder weniger abgesenkt.

Die Kurzwegdestillation ist das bevorzugte Verfahren zur Abtrennung von überschüssigem Diisocyanat, und sie wird in der Regel bei Temperaturen von 100 bis 160°C und einem Druck von 0,01 bis 3 mbar durchgeführt. Der Restmonomergchalt beträgt danach bevorzugt weniger als 1 Gew.-%, besonders bevorzugt weniger als 0,5 Gew.-% (Diisocyanat).

Die gesamten Verfahrensschritte können gegebenenfalls in Abwesenheit inerter Lösungsmittel durehgeführt werden. Als inerte Lösungsmittel sind dabei solche zu verstehen, die unter den gegebenen Reaktionsbedingungen nicht mit den Edukten reagieren. Beispiele sind Ethylacetat, Butylacetat, Methoxypropylacetat, Methylethylketon, Methylisobutylketon, Toluol, Xylol, aromatische oder (cyclo-)aliphatische Kolllenwasserstoffgemische oder beliebige Gemische derartiger Lösungsmittel. Bevorzugt werden die erfindungsgemäßen Umsetzungen jedoch lösemittelfrei durchgeführt.

Die Zugabe der beteiligten Komponenten kann sowohl bei der Herstellung der isocyanatgruppenhaltigen Prepolymere als auch bei Allophanatisierung in beliebiger Reihenfolge erfolgen. Bevorzugt ist jedoch die Zugabe des Polyetherpolyols b) zum vorgelegten Hexamethylendiisocyanat der Komponenten a) und c) und schließlich die Zugabe des Allophantisierungs-Katalysators d).

In einer bevorzugten Ausführungsform der Erfindung wird das Hexamethylendiisocyanate der Komponenten a) und c) in einem geeigneten Reaktionsgefäß vorgelegt und, gegebenenfalls unter Rühren, auf 40 bis 100 °C erwärmt. Nach Erreichen der gewünschten Temperatur werden unter Rühren dann die Polyhydroxyverbindungen der Komponente b) zugegeben und solange gerührt, bis der theoretische NCO-Gehalt des nach der gewählten Stöchiometrie zu erwarfenden Polyurethan-Prepolymers erreicht oder geringfügig unterschritten ist. Jetzt wird der Allophanatisierungs-Katalysator d) zugegeben und die Reaktionsmischung solange auf 50 und 100 °C erwärmt, bis der gewünschte NCO-Gehalt erreicht oder geringfügig unterschritten ist. Nach Zugabe von sauren Additiven als Stabilisatoren wird das Reaktionsgemisch abgekühlt oder direkt der Kurzwegdestillation zugeführt. Dabei wird das überschüssige Hexamethylendiisocyanat bei Temperaturen von 100 bis 160 °C und einem Druck von 0,01 bis 3 mbar bis auf einen Restmonomergehalt von weniger als 1 %, bevorzugt weniger als 0,5 %, abgetrennt- Nach der Kurzwegdestillation kann gegebenenfalls weiterer Stabilisator zugegeben werden.

Die in dem beanspruchten Verfahren gebildeten Allophanate entsprechen typischerweise der allgemeinen Formel (1), 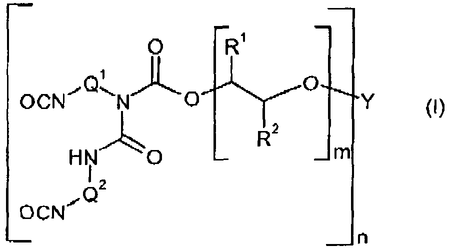 worin
- Q¹ und Q²: -(CH₂),
- R¹ und R²: unabhängig voneinander für Wasserstoff oder einen C₁-C₄-Alkylrest stchen, wobei R¹ und R² bevorzugt Wasserstoff und/oder Methylgruppen sind,
- Y: der Rest eines Starter-Moleküls der genannten Art mit einer Funktionalität von 2 bis 6 ist, und somit
- n: für einen Wert von 2 bis 6 steht, welche durch Verwendung von verschiedenen Starter-Molekülen selbstverständlich auch keine ganze Zahl sein muss, sowie
- m: bevorzugt so vielen Monomereinheiten entspricht, dass das zahlenmittlere Molekulargewicht des der Struktur zugrunde liegenden Polyethers 100 bis 300 g/mol beträgt.

Vorzugsweise werden Allophanate erhalten, welche der allgemeinen Formel (II) entsprechen, 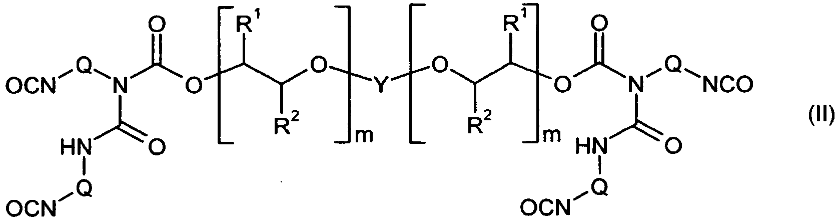 worin
- Q: -(CH₂)₆-,
- R¹ und R²: unabhängig voneinander für Wasserstoff oder für einen C₁-C₄-Alkylrest stehen, wobei R¹ und R² bevorzugt Wasserstoff und/oder Methylgruppen sind,
- Y: für den Rest eines difunktionellen Starter-Moleküls der genannten Art steht und
- m: so vielen Monomereinheiten entspricht, dass das zahlenmittlere Molekulargewicht des der Struktur zugrunde liegenden Polyethers 100 bis 300 g/mol beträgt.

Die erfindungsgemäß hergestellten Allophanate haben typischerweise zahlenmittlere Molekulargewichte von 700 bis 1000 g/mol.

Die erfindungsgemäß hergestellten Allophanate haben typischerweise Viskositäten bei 23 °C von 500 bis 4000 mPas.

Die nach dem erfindungsgemäßen Verfahren erhältlichen Produkte zeichnen sich durch ihre hohe Funktionalität, ihren hohen NCO-Gehalt, ihre niedrige Viskosität und ihre gute Viskositätsstabilität aus. Die Viskositätszunahme nach 28 Tagen Lagerung bei 50 °C beträgt bevorzugt weniger als 30 %

Die Allophanate können beispielsweise zur Herstellung von Polyurethanen, Polyharnstoffen oder Polyurethanharnstoffen eingesetzt werden, indem man sie mit geeigneten Polyolen bzw. Polyaminen oder auch einer Mischung aus beiden zur Reaktion bringt. Die Umsetzung kann dabei bei Raumtemperatur oder darunter, aber auch bei erhöhten Temperaturen erfolgen (Einbrennen). Die so erhaltenen Polyurethane bzw. Polyharnstoffe sind besonders geeignet als Beschichtungen, Klebstoffe und Dichtstoffe.

Gegenstand der Erfindung ist daher auch die Verwendung der erfindungsgemäß hergestellten Hexamethylendiisocyanat-Prepolymere mit Allophanat-Struktureinheiten in Klebstoff-Formulierungen oder Dichtstoff-Formulierungen.

Diese Formulierungen können beispielsweise mit Metallen, Kunststoffen, Keramik, Glas sowie Naturstoffen in Kontakt gebracht werden, wobei die genannten Substrate zuvor einer gegebenenfalls notwendigen Vorbehandlung unterzogen worden sein können.

### Beispiele

Sofern nicht abweichend erwähnt sind alle Prozentangaben als Gewichtsprozent zu verstehen.

Die Bestimmung der NCO-Gehalte erfolgte durch Rücktitration von im Überschuss zugesetztem Di-n-butylamin mit Salzsäure.

Die Viskositäten wurden mit einem Rotationsviskosimeter der Firma Haake bei 23°C bestimmt.

Die Farbzahl wurde nach DIN EN 1557 (Hazen) ermittelt.

### Beispiel 1

Zu 11581 g (68,85 Mol) 1,6-Hexamethylenndiisocyanat (HDI) wurden zunächst 30 ppm Isophtaloyldichlorid gegeben, danach wurde die Mischung unter Rühren auf 100 °C erwärmt. Nun wurden innerhalb von ca. 3 Stunden 1000 g (4,59 Mol) eines Polypropylenglycols zugegeben, (OH-Zahl 515 mg KOH/g). Die Reaktionsmischung wurde danach solange auf 100 °C erwärmt, bis ein NCO-Gehalt von 42,6 Gew.-% erreicht war. Nun wurde die Temperatur auf 90 °C vermindert und die Reaktionsmischung nach Zugabe von 205 ppm Zink(II)bis(2-ethylhexanoat) solange gerührt, bis der NCO-Gehalt bei 36,9 Gew.-% lag (ca. 4 Stunden). Nach Zugabe von 230 ppm Isophtaloyldichlorid wurde das überschüssige 1,6-Hexamethylendiisocyanat (HDI) bei ca. 0,1 mbar und 140 °C mittels Dünnschichtdestillation entfernt.

Die Allophanatisierung verlief nahezu vollständig und es wurde ein klares, farbloses Produkt mit einem NCO-Gehalt von 17,6 Gew.-% und einer Viskosität von 3260 mPas (23°C) bei einem HDI-Restmonomergehalt von 0,05 Gew.-% erhalten.

| **Bestimmung der Lagerstabilität** | |
|---|---|
| Lagerzeit [d] bei 50 °C | Viskosität bei 25 °C [mPas] |
| 0 | 2180 |
| 1 | 2257 |
| 3 | 2269 |
| 7 | 2330 |
| 14 | 2443 |
| 28 | 2612 |

| **Klebungen** | |
|---|---|
| Substrat: Buche / Buche | |
| Aushärtezeit [h] | Zugscherfestigkeit [N/mm²] |
| 48 | 4,33 |
| 168 | 10,13 |
| 336 | 11,36 |

## Patentansprüche

1. Verfahren zu Herstellung von Hexamethylendiisocyanat-Prepolymeren mit Allophanat-Struktureinheiten, **dadurch gekennzeichnet, dass**
A) zunächst in Abwesenheit eines Katalysators in einem molaren NCO-Gruppen- zu OH-Gruppen-Überschuss von 10:1 bis 20:1
a) Hexamethylendiisocyanat mit
b) einer oder mehreren Polyhydroxyverbindungen eines zahlenmittleren Molekulargewicht Mn von 100 bis 300 g/mol
zu einem NCO-funktionellen Polyurethan-Prepolymer umgesetzt werden, und dessen Urethangruppen werden anschließend unter weiterer Umsetzung mit
c) dem überschüssigen Hexamethylendiisocyanat und
d) Zink(II)-octoat als Katalysator
teilweise oder vollständig allophanatisiert, der Katalysator wird anschließend mit Isophthaloyldichlorid gestoppt und durch eine abschließende Kurzwegdestillation wird der Restmonomerengehalt des so erhaltenen Hexamethylendiisocyanat-Prepolymeren mit Allophanat-Struktweinheiten auf 0,5 Gew.-% oder weniger abgesenkt, so dass
B) der NCO-Gehalt des Hexamethylendiisocyanat-Prepolymeren mit Allophanat-Struktureinheiten zwischen 15 und 24 Gew.-% NCO-Gruppen liegt und
C) die Viskosität des Hexamethylendiisocyanat-Prepolymeren mit Allophanat-Struktureinheiten zwischen 500 und 4000 mPas bei 23 °C liegt.

2. Verwendung der gemäß Anspruch 1 erhaltenen Hexamethylendiisocyanat-Prepolymeren mit Allophanat-Struktureinheiten in Klebstoff-Formulierungen.

3. Verwendung der gemäß Anspruch 1 erhaltenen Hexamethylendiisocyanat-Prepolymeren mit Allophanat-Struktureinheiten in Dichtstoff-Formulierungen.

## Claims

1. Method for preparing hexamethylene diisocyanate prepolymers having allophanate structural units, **characterized in that**
A) initially in the absence of a catalyst and in a molar excess of NCO groups to OH groups of 10:1 to 20:1
a) hexamethylene diisocyanate and
b) one or more polyhydroxy compounds having a number average molecular weight Mn of 100 to 300 g/mol.
are reacted to give an No-functional polyurethane prepolymer, the urethane groups of which are subsequently, by further reaction with
c) the excess hexamethylene diisocyanate and
d) zinc(II) octoate as catalyst,
partially or completely allophanatised, the catalyst then being deactivated with isophthaloyl dichloride and, by means of a final short-path distillation, the remaining monomer content of the hexamethylene diisocyanate prepolymer having allophanate structural units thus obtained being reduced to 0.5% by weight or less, such that
B) the NCO content of the hexamethylene diisocyanate propolymer having allophanate structural units is between 15 and 24% by weight of NCO groups and
C) the viscosity of the hexamethylene diisocyanate prepolymer having allophanate structural units is between 500 and 4000 mPa at 23°C.

2. Use of the hexamethylene diisocyanate prepolymers having allophonate structural units obtained according to Claim 1 in adhesive formulations.

3. Use of the hexamethylene diisocyanate prepolymers having allophonate structural units obtained according to Claim 1 in sealant formulations.

## Revendications

1. Procédé pour la production de prépolymères d'hexaméthylènediisocyanate à motifs structuraux allophanate, **caractérisé en ce que**
A) d'abord on fait réagir en absence d'un catalyseur, en un excès molaire des groupes NCO par rapport aux groupes OH de 10:1 à 20:1
a) de l'hexaméthylènediisocyanate avec
b) un ou plusieurs composés polyhydroxylés ayant une masse moléculaire moyenne en nombre Mn de 100 à 300 g/mole
pour obtenir un prépolymère polyuréthane à fonction NCO, et dont les groupes uréthane sont ensuite partiellement ou totalement allophanatisés par une nouvelle réaction avec
c) l'hexaméthylènediisocyanate en excès et
d) de l'octoate de zinc (II) en tant que catalyseur,
ensuite on arrête le catalyseur à l'aide de dichlorure d'isophtaloyle et, par une distillation finale à trajet court, on abaisse à 0,5 % en poids ou moins la teneur en monomères résiduels du prépolymère hexamethylènediisocyanate à motifs structuraux allophanate ainsi obtenu, de sorte que
B) la teneur en NCO du prépolymère d'hexaméthylène-diisocyanate à motifs structuraux allophanate est comprise entre 15 et 24 % en poids de groupes NCO et
C) la viscosité du prépolymère d'hexaméthylène-diisocyanate à motifs structuraux allophanate est comprise entre 500 et 4 000 mPa·s à 23 °C.

2. Utilisation du prépolymère d'hexaméthylène-diisocyanate à motifs structuraux allophanate obtenu selon la revendication 1, dans des formulations d'adhésifs.

3. Utilisation du prépolymère d'hexaméthylène-diisocyanate à motifs structuraux allophanate obtenu selon la revendication 1, dans des formulations de matières d'étanchéité.
